# FASCICULE DE BREVET EUROPEEN

(11) **EP 2 416 794 B1**
(45) Date de publication et mention de la délivrance du brevet: **25.12.2013**
(21) Numéro de dépôt: 09784375.9
(22) Date de dépôt: 10.04.2009
(51) Int. Cl.: A61K 36/71, A61K 45/06, A61K 31/05, A61K 31/353, A61K 35/02, A61K 36/74, A61K 36/73, A61P 31/12

(54) **COMPOSITION À BASE DE PLANTES POUR LE TRAITEMENT OU LA PRÉVENTION DE MALADIES VIRALES DU SANG CAUSÉES PAR LE VIRUS DE L' IMMUNODÉFICIENCE HUMAINE (VIH) OU L'HÉPATITE C**
PFLANZLICHE ZUSAMMENSETZUNG ZUR BEHANDLUNG ODER PROPHYLAXE VON VIRALEN BLUTÜBERTRAGENEN KRANKHEITEN DURCH DAS HUMANE IMMUNSCHWÄCHEVIRUS (HIV) ODER HEPATITIS C
PLANT COMPOSITION FOR THE TREATMENT OR PREVENTION OF VIRAL BLOOD-BORNE DISEASES CAUSED BY THE HUMAN IMMUNODEFICIENCY VIRUS (HIV) OR HEPATITIS C

(43) Date de publication de la demande: 15.02.2012
(73) Titulaire: El Khatib, Nadia, Beyrouth (LB); El Kettany, Sleimen, 78430 Louveciennes (FR)
(72) Inventeur: EL KHATIB ,Nadia., Beyrouth (LB); EL KETTANY, Sleimen., Chedid center bldg, floor 5 (LB)
(74) Mandataire: Thinat, Michel
(86) Numéro de dépôt international: PCT/FR2009/050678
(87) Numéro de publication internationale: WO 2010/116048

(56) Documents cités:
- EP-A1- 0 307 616
- FR-A1- 2 296 426
- FR-A1- 2 622 800
- FR-A1- 2 901 699
- US-A- 202 932

## Description

L'invention concerne une composition à base de plantes pour le traitement ou la prévention de maladies virales du sang, telles que les maladies causées par le virus d'immunodéficience humaine (VIH) ou l'hépatite C.

Les porteurs du VIH développent tardivement les symptômes liés à la maladie puisque ceux-ci ne surviennent qu'au bout de deux à dix ans suivant leur contamination. Durant ce délai, comme aucun effet de la maladie n'est visible, les porteurs peuvent transmettre le virus sans le savoir lors de rapports sexuels non protégés, par contamination sanguine ou par transmission de la mère à l'enfant. Cette période d'incubation longue est ainsi en partie responsable d'une expansion épidémique de la maladie.

En outre à ce jour, il n'existe aucun vaccin permettant de se protéger du VIH et les traitements connus disponibles, tels que les trithérapies rétrovirales, ont une certaine efficacité mais ne permettent aucune guérison.

De plus, ils ne sont généralement pas prescrits au début de la séropositivité car ils présentent de nombreux effets secondaires indésirables, ainsi qu'une certaine toxicité.

Parmi les effets secondaires les plus fréquemment rencontrés, et les moins graves, on compte les maux de tête, les nausées et vomissements, la fatigue, la perte d'appétit, les accès de fièvre, les picotements ou brûlures aux mains et aux pieds, les diarrhées et les problèmes de peaux.

De nombreux effets secondaires sont persistants et deviennent de plus en plus invalidants du fait de leur durée. Lorsque les effets passagers cités précédemment perdurent, ils peuvent devenir irréversibles et très handicapants.

D'autres effets secondaires graves n'apparaissent qu'avec le temps ; ils tiennent généralement à la toxicité des molécules.

Et certains effets secondaires sont mortels :
- les hypersensibilités cutanées ou respiratoires dues à l'abacavir (Ziagen®) ;
- les hypersensibilités cutanées et intolérance hépatique dues à la névirapine (Viramune®) ;
- les pancréatites aiguës dues à la ddI (Videx®) ;

Les traitements classiques connus présentent donc des inconvénients non négligeables.

Les documents FR 2 296 426, FR 2 622 800 et US 202 932 décrivent des compositions à base de plantes comprenant de la fleur de soufre pour le traitement de maladies virales autres que celles causées par le virus de l'immunodéficience humaine ou de l'hépatite C.

L'invention propose de résoudre ce problème et a pour objet une composition pour le traitement ou la prévention de maladies virales du sang causées par le virus VIH ou l'hépatite C, efficace en terme de diminution de la charge virale d'un patient et d'augmentation de lymphocytes T de sous population CD₄, et qui génère moins d'effet secondaire chez les patients l'ayant ingérée voire aucun.

A cet effet, l'invention concerne une composition à base de plantes pour le traitement ou la prévention de maladies virales du sang causées par le virus de l'immunodéficience humaine (VIH) ou l'hépatite C.

Selon l'invention, la composition comprend de la fleur de soufre, au moins une plante contenant des agents tannins et de la catéchine choisie parmi l'Aigremoine Eupatoire (GAFT) ou le Gambier (Uncaria gambir), ainsi qu'un support pharmaceutiquement acceptable.

Selon encore une autre caractéristique, la composition comprend deux plantes différentes contenant des agents tannins et de la catéchine.

Dans ce cas, ces plantes sont l'Aigremoine Eupatoire (GAFT) ou le gambier (Uncaria gambir)

Avantageusement, la composition comprend en outre de la Nigelle.

Plus précisément, la Nigelle est présente sous la forme d'huile.

En outre, la composition peut comprendre un agent antiputride.

De préférence, l'agent antiputride est le menthol.

Selon une autre caractéristique, la composition comprend un agent d'enrobage.

Cet agent d'enrobage est plus particulièrement le miel.

La composition selon l'invention telle que définie ci-dessus provient ainsi uniquement de matériaux naturels, d'essence de plantes et de minéraux extraits de la terre, mélangés dans des proportions prédéterminées. Il a été prouvé par des tests expérimentaux que ce produit n'a aucun effet secondaire de quelque sorte que ce soit et ne contient aucun stimulant sédatif ou tranquillisant.

Le procédé de fabrication de la composition ci-dessus comprend une étape de mélange d'au moins une plante contenant des agents tannins et de la catéchine, avec de la fleur de soufre, et un support pharmaceutiquement acceptable.

La plante contenant de la catéchine et des agents tannins est choisie parmi l'Aigremoine Eupatoire (GAFT) ou le gambier (Uncaria gambir).

Selon une variante intéressante, deux plantes différentes contenant des agents tannins et de la catéchine sont utilisées, ces plantes étant l'Aigremoine Eupatoire (GAFT) et le gambier (Uncaria gambir).

Avantageusement, ce procédé comprend une étape d'ajout d'huile de Nigelle.

De préférence, l'Aigremoine Eupatoire (GAFT) est utilisée sous la forme de jus obtenu par distillation.

Le gambier est préférentiellement utilisé sous la forme de poudre finement moulue.

Selon une autre caractéristique, le procédé comprend une étape préliminaire de dissolution de cristaux de menthol dans de l'alcool pur.

De préférence, le support pharmaceutiquement acceptable comprend du miel d'une viscosité importante.

Ce procédé, est simple de mise en oeuvre puisqu'il comprend principalement une étape de mélange de jus d'Aigremoine Eupatoire (GAFT), de fleur de soufre, d'huile de Nigelle, de gambier, avec un support pharmaceutiquement acceptable qui est du miel.

L'invention sera mieux comprise et d'autres buts, avantages et caractéristiques de celle-ci apparaîtront à la lecture de la description suivante, faite en référence aux figures annexées parmi lesquelles :
- le tableau 1 reprend les valeurs de charge virale relevées sur trois patients auxquels a été administrée la composition selon l'invention,
- le graphique 1 représente l'évolution de la charge virale du premier patient auquel a été administré la composition selon l'invention, en fonction du temps,
- le graphique 2 représente l'évolution de la charge virale du second patient auquel a été administré la composition selon l'invention, en fonction du temps,
- le graphique 3 représente l'évolution de la charge virale du troisième patient auquel a été administré la composition selon l'invention, en fonction du temps,
- le tableau 2 reprend les valeurs de lymphocytes T (CD4) relevées sur une période de 200 jours sur les trois patients susmentionnés,
- les graphiques 4 à 6 représentent l'évolution des CD4 en fonction du temps pour respectivement ces trois patients.

L'invention sera présentée dans une première partie par une liste des ingrédients principaux la constituant, dans une deuxième partie par le procédé de sa fabrication à partir de ces ingrédients, par la suite par son mode d'administration à un patient et par son efficacité telle que révélée par les tests cliniques pratiqués sur des patients et confirmés. Enfin, le détail de chaque ingrédient est précisé dans une dernière partie.

### Ingrédients constitutifs

La composition selon l'invention comprend les ingrédients listés dans le tableau ci-dessous, et idéalement dans les proportions qui y sont mentionnées :

| Ingrédients : | masse dans la composition testée ( g ) | Pourcentage massique dans la composition testée sur des patients (% massique) | Plage de pourcentages massiques pouvant permettre d'assurer l'efficacité de la composition (% massique) | Fonction(s) de l'ingrédient considéré isolément |
|---|---|---|---|---|
| GAFT (nom latin : Agrimonia Eupatoria, nom français : Aigremoine Eupatoire) | 5 | 0,54 | 0,54 = X < 1,05 | deux formes de catéchines + et -, la + catéchine a un effet antibiotique et une action préventive à la formation des radicaux libres |
| Uncaria Gambir, nom français : gambier | 4 | 0,43 | 0,43 < X < 1 | anti-inflammatoire, anti hémorragique |
| Huile de Nigella Sativa (nom latin : Nigella Sativa L., nom français : Huile de Nigelle ou Huile végétale de cumin noir ) | 5 | 0,54 | 0,54 = X < 0,87 | analgésique, antiseptique, expectorant, antiseptique (contre les bactéries ou organismes à gram positive et gram négative), anti-colique, inhibe l'histamine, agit comme un bronchodilatateur |
| Fleur de soufre comestible (ou soufre sublime) | 2 | 0,22 | 0,19 < X < 0,27 | action énergétique au niveau du foie |
| Cristaux de menthol | 1,5 | 0,16 | 0,15 < X < 0,18 | antiputride |
| Alcool pur (Ethanol 95°) | 4 | 0,43 | 0,38 < X < 0,49 | dissolvant (du menthol et des huiles de Nigella Sativa) |
| Miel pur | 900 | 97,67 | 92.24 < X < 99.83 | enrobe la composition, effet antioxydant, facilite l'absorption par l'estomac |
| Total | 921,50 | 100,00 | | |

### Procédé de préparation

Ces ingrédients sont associés selon le procédé suivant :
1.5 Grammes de MENTHOL-CRISTAL est disposé au sein d'un récipient, à température et pression ambiantes.
4 grammes d'alcool pur, de préférence de l'éthanol à 95°, sont introduites dans le récipient afin de dissoudre les cristaux de menthol et, lorsque celle ci aura été ajoutée, l'huile de Nigelle.

L'ensemble est mis au repos 5 minutes, puis remué pour aider la dissolution.
2 grammes de fleur de souffre comestible sont incorporées au mélange obtenu.
5 grammes de jus de GAFT sont ensuite ajoutées. Ce jus est préalablement préparé en utilisant 1 kg de feuilles et de tiges de GAFT séchées, trempées dans 3.5 litres d'eau à température ambiante pendant 20 heures. Ce mélange subit ensuite un processus de distillation en étant chauffé à feu doux et en collectant la vapeur formée dans un récipient froid pour recueillir environ 2 litres d'essence de GAFT ou jus de GAFT.

L'alcool, le menthol, la fleur de soufre et le jus de GAFT sont ensuite mélangés avec 900 grammes de miel pur, d'une viscosité importante afin de faciliter l'absorption au niveau de l'estomac.
4 grammes d'UNCARIA GAMBIR sont moulues finement et incorporées au mélange.
5 grammes d'huile de Nigelle (NIGELLA SATIVA OIL ou HABAT EL BARAKAT), extraite à froid, sont incorporées au mélange, et dissoutes dans celui-ci.

### Mode d'administration

### Par voie orale

Environ 20 grammes de composition toutes les 6 heures, quatre fois par jour, pendant au moins 12 mois, les effets de la composition sur l'organisme étant oberservés dès 40 jours après le début du traitement.

### Efficacité dans le cadre du traitement de patients atteints du VIH

### Première patiente

Dans un premier temps, la composition a été utilisée sur un patient atteint du VIH et ayant un ulcère gastrique en 2005, ce patient étant une femme originaire de Cuba et d'environ 60 ans. A cette époque, elle était à une phase de la maladie où elle développait tous les symptômes de celle-ci et souffrait en plus d'un ulcère gastrique sévère.

La composition lui avait été donnée dans l'objectif de guérir son ulcère et de façon surprenante, après cinq mois de traitement, et alors que la patiente était sur le point de faire le test standard préliminaire à la prise du traitement classique (trithérapie), il a été découvert que non seulement l'ulcère était entièrement guéri mais également que son taux de CD4 était égal à 635 unités par mm³ de sang (sachant qu'avant le traitement il était inférieur à 500 unités par mm³ de sang) et la charge virale presque indétectable, rendant inutile le commencement du traitement classique.

La patiente mentionnée ci-dessus vit, suite à la prise de la composition selon l'invention, en bonne santé à Cuba depuis juin 2005 sans aucun traitement supplémentaire d'aucune sorte.

### Les cinq patients suivants

Cinq autres patients séropositifs d'origine ukrainienne ont été traités avec la composition selon l'invention.

Leur état de séropositivité a été détecté en 1997 et en 1998 ; trois d'entre eux étaient frappés en outre par l'hépatite C avant de suivre le traitement selon l'invention, ces cinq patients étaient suivis de près par leur médecin traitant.

Un examen préliminaire de leur santé clinique a été effectué et tous présentaient les symptômes d'anxiété, de fièvre, de douleurs musculaires, de douleurs au foie, de fatigue, de nausées, de vomissements, de pertes d'appétit, et de maux de tête.

Différents échantillons de sang ont été analysés par des laboratoires à différentes dates depuis le début du traitement jusqu'à 70 jours après. Trois examens cliniques et histologiques ont été effectués premièrement à l'arrivée des patients, un mois après le traitement, et deux mois après le traitement.

Après dix jours de traitement, tous les symptômes précédemment cités desquels souffraient les patients qui étaient à la deuxième phase de la maladie, avaient disparu.

Tous les patients étaient de façon évidente en bonne santé, ne soufraient plus d'hépatite C, avaient vu croître leur volume corporel, ne présentaient aucune fièvre, et ont constaté une réapparition de leur désir sexuel, qui était pourtant quasiment inexistant avant le traitement.

Des améliorations similaires ont été observées avec les résultats de tests hématologiques. Les mesures de CD4 et de charge virale ont été effectuées à différentes étapes du traitement et une légère amélioration du taux de CD4 a été observée. De façon analogue, les résultats relatifs à la charge virale furent satisfaisants.

### Les trois derniers patients

Des tests cliniques supplémentaires ont été effectués sur trois autres patients en collaboration avec un centre international de recherche sur le VIH reconnu, situé à Paris.

Ainsi, à la mi-août 2007, trois patients porteurs du VIH et originaires du Liban, plus précisément deux hommes et une femme qui avaient été infectés les uns par les autres, ont été approchés. Les deux hommes vivaient le premier stade de la maladie, alors que la femme de 26 ans avait déjà été convoquée par son médecin traitant afin de commencer le traitement classique (trithérapie).

Les trois patients ont décidé de suivre scrupuleusement la méthode de traitement développée pour la composition selon l'invention sur recommandation du centre de recherches précité, la femme ayant en outre refusé le traitement classique.

Avant de commencer le traitement avec la composition selon l'invention, une première prise de sang a été effectuée sur les trois patients (« jour 0 » des tableaux 1 et 2). Les résultats ont été communiqués au centre de recherches précité.

L'ensemble des trois patients a commencé à prendre la composition selon l'invention les 19 et 25 août 2007, en ingérant chaque jour, par voie orale 20 grammes de la composition 3 à 4 fois par jour et à environ six heures d'intervalle.

Une deuxième prise de sang a été effectuée en octobre le 4 de l'année 2007 et a été directement envoyée au centre de recherches susmentionné pour analyse, évaluation et comparaison avec les résultats de tests précédents.

Les résultats furent surprenants puisque, tels que visibles sur le tableau n° 1, au bout de 40 jours, la charge virale de l'ensemble des patients a diminué de façon drastique de 76% pour le premier patient, 88% pour le second et 92% pour le troisième.

Compte-tenu de ces résultats positifs, les trois patients ont continué à prendre régulièrement la composition selon l'invention pendant encore 160 jours, portant ainsi à 200 jours la durée totale du traitement, tel que mentionné sur les tableaux 1 et 2.

Les résultats obtenus d'après les prises de sang confirment l'efficacité de la composition dans le temps :
Pour un même patient, par exemple le patient n° 1, la baisse de charge virale est constante du début à la fin du traitement et cette charge virale s'abaisse jusqu'à 95% en toute fin de traitement (200^{ème} jour).

Concernant le patient n° 2, on observe également une baisse drastique entre le début de traitement et le 40^{eme} jour de traitement puisque le nombre de copies de virus par ml de sang baisse de 88% pour ensuite, à partir du 40^{ème} jour, se situer toujours en dessous de 12 000 copies et finir autour de 8 000 copies par ml de sang, ce qui représente par rapport au début de traitement une diminution de 83% de la charge virale.

Il en est de même pour le patient 3 avec, comme visible sur le graphique 2, une baisse drastique de 92% de la charge virale les 40 premiers jours puis une stabilisation en dessous de 20 000 copies par ml de sang entre les 40 et 200 jours de traitement, pour finir à un taux bas autour de 5000 copies par ml de sang, ce qui représente une baisse de 94% par rapport au tout début de traitement.

En ce qui concerne l'évolution du nombre de lymphocytes T par mm³ de sang, on observe que ceux-ci sont en légère augmentation entre le début et la fin du traitement pour le patient n° 1 puisque l'on mesure une augmentation de 17% de ces lymphocytes entre le jour 0 et le 200^{ème} jour de traitement. On observe une augmentation beaucoup plus nette concernant les patients n° 2 et 3 puisque entre le début de traitement et la fin de celui-ci pour le patient n° 2 le nombre de lymphocytes T par mm³ de sang a augmenté de 86%, et de 78% pour le patient n° 3, avec une remarquable augmentation au bout du 144^{ème} jour de 102% puisque les lymphocytes du patient 3 atteignent 930 espèces par mm³ de sang à cet instant.

Il est important de noter que l'ensemble des patients mentionnés ci-dessus étaient et sont encore sous surveillance médicale de docteurs spécialisés et que cette surveillance confirme leur bonne santé. En outre, aucun effet secondaire néfaste n'a été observé.

D'après les recherches effectuées, l'effet de la composition selon l'invention tient à son action drastique sur le virus lui-même.

Au fur et à mesure des tests pratiqués sur les patients, la composition, son mode d'administration et son procédé de fabrication ont été optimisés.

De cette phase d'optimisation, il ressort que le GAFT et la fleur de soufre sont les éléments de base pour obtenir, lorsque combinés l'un à l'autre, un effet de diminution de la charge virale et d'augmentation des CD4.

Dans le GAFT, on trouve des agents tannins et de la catéchine (*cf* paragraphe « GAFT » du chapitre « *Détails sur les ingrédients principaux constitutifs de la composition* »).

Egalement, dans le gambier (Uncaria Gambir), on retrouve des agents tannins et de la catéchine.

Le gambier, contenant des agents tannins et de catéchine pourrait donc former, avec la fleur de soufre et à la place du GAFT, les éléments de base pour obtenir, lorsque combinés l'un à l'autre, un effet de diminution de la charge virale et d'augmentation des CD4.

Egalement, d'autres plantes contenant de la catéchine et des agents tannins, telles que celles mentionnées dans la partie suivante détaillant les ingrédients utilisés, peuvent former avec la fleur de soufre les éléments de base pour obtenir, lorsque combinés l'un à l'autre, un effet de diminution de la charge virale et d'augmentation des CD4.

L'huile de Nigelle est également un élément constitutif de la composition ayant une participation importante dans l'effet de diminution de la charge virale et d'augmentation des CD4.

Les compositions contenant des plantes GAFT et gambier, en combinaison avec l'huile de Nigelle et la fleur de soufre, se sont révélées être les plus satisfaisantes en terme d'effet de diminution de la charge virale et d'augmentation des CD4.

La composition selon l'invention s'est donc révélée efficace dans le traitement de patients atteints par le virus du VIH et par l'hépatite C.

Elle est obtenue à partir de plantes et de minéraux ce qui lui évite de causer des effets secondaires néfastes.

Son procédé de fabrication est en outre simple de mise en oeuvre.

Bien entendu, aucun des tests pratiqués n'a été établi en divulguant à quiconque ni les ingrédients constitutifs de la composition ni son procédé de fabrication. En effet, les patients ingéraient immédiatement après l'avoir reçue, la composition selon l'invention en présence de l'équipe médicale.

On donne dans ce qui suit le détail des ingrédients constitutifs de la composition selon l'invention.

**Détails sur les ingrédients principaux constitutifs de la composition**

### 1) GAFT ou Aigremoine Eupatoire ou Agrimonia Eupatoria

Nom scientifique :
   Agrimonia eupatoria L
Famille :
   Rosaceae
   Autres appellations : Eupatoire des anciens, Herbe de Saint Guillaume, agrimone, Herbe de Ste Madeleine
Botanique et géographie
   Origine : Haies, bois et terrains de toute l'Europe, Afrique du Nord, Asie occidentale et septentrionale.
   Description botanique : - Plante herbacée vivace de 40 à 60 cm de haut, à tige velue rougeâtre - Grandes feuilles, divisées en segments inégaux - Fleurs petites, jaunes, très nombreuses, groupées en longues grappes terminales - Fruit : 1 à 2 akènes enfermés dans un calice hérissé
Parties utilisées :
   Feuilles et sommités fleuries
Constituants :
   Tannins, Coumarines, Flavan-3-ol (constitué par des catéchines notamment), quercétine, kaempferole, luteolin, apigenin, différents acides phénoliques, vitamines B et K
Risques pour homme :
   Vraisemblablement aucun

### 2) Gambier ou Gambir ou Uncaria Gambir

Règne : Plantae
Sous-règne : Tracheobionta
Division : Magnoliophyta
Classe : Magnoliopsida
Sous-classe : Asteridae
Ordre : Rubiales
Famille : Rubiaceae
Genre : Uncaria
Nom binominal : Uncaria gambir (W.Hunter) Roxb., 1824 Ordre : Gentianales

### Utilisation :

Le gambier est une plante médicinale aux propriétés toniques et astringentes que l'on utilise pour traiter, entre autres, les brûlures, la diarrhée, la toux, les maux de gorge et les ulcères. La plante contient de l'acide tannique et de la catéchine.

### 2.1 L'acide tannique

Cet acide peut être obtenu par dégradation des tanins par des microorganismes.

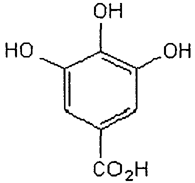

Les tanins sont des combinaisons complexes de glucose et d'acide gallique que l'on trouve dans l'écorce de chêne, l'écorce de châtaigner et dans les noix de galle. Les tanins rendent les peaux imputrescibles d'où leur utilisation dans le traitement des cuirs. On les utilise aussi dans la fabrication des encres.

### 2.2 Catéchine (catechin en anglais):

La catéchine est une molécule de la famille des flavonoïdes de la sous-classe des flavanols. Elle est aussi connue sous le nom de catéchol.

Initialement découverte dans les fruits de l'acacia à cachou (Acacia catechu) duquel elle dérive son nom, la catéchine et ses nombreux isomères sont de puissants antioxydants qui aident à prévenir les maladies inflammatoires et coronariennes.

La catéchine est une molécule chirale qui présente deux formes symétriques : la (+)-catéchine et la (-)-catéchine. Pour éviter la confusion entre la classe et le composé, ce dernier est désigné dans ce contexte par (+/-)-catéchine.

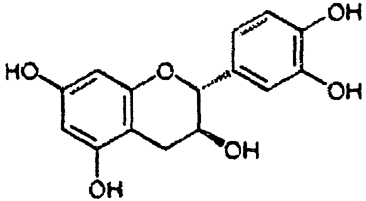

La (+)-catéchine est un antibiotique et un antioxydant qui évite la formation de radicaux libres.

D'autres plantes que le gambier sont riches en catéchines et en acides tannins :
- le théier (Camellia sinensis) : les flavonoïdes de la feuille de théier sont constitués à 80% de flavanols, à savoir (+)-catéchine C, (-)-épicatéchine EC, (+)-gallocatéchine GC et leur esters galliques.
   Les thés verts, oolong et noirs se distinguent par l'importance de l'oxydation enzymatique subies par les feuilles lors d'un processus improprement appelé « fermentation ». Les flavonoïdes du thé vert, comme ceux de la feuille fraiche, sont à 80% des flavanols mais après oxydation, il n'en reste plus que 20 à 30% dans le thé noir. Le thé est connu pour être riche en agents tannins.
- la vigne (Vitis vinifera) : le raisin est riche en (+)-catéchine, (-)-épicatéchine et son ester gallique, l'épicatéchine-3-gallate. Ces flavanols sont concentrés dans les pépins, à des doses diverses suivant le cépage, le terroir, le millésime, la maturité phénolique. On trouve des concentrations élevées dans les pépins de raisin Merlot et Cabernet Sauvignon. Le raisin est riche en agents tannins.
- le cacaoyer (Theobroma cacao) : la fève de cacao contient de 12 à 18% de polyphénols (en % de matières sèches) avec environ 35% de ceux-ci sous forme de (-)-épicatéchine (pour la fève de Forastero non fermentée). Pour devenir du cacao, les fèves doivent subir une fermentation, un séchage et une torréfaction. Durant ces nombreuses opérations la majeure partie des catéchines et procyanidols sont convertis en quinones. Dans le cacao, les flavanols restent cependant majoritaires parmi les polyphénols, avec en premier la (-)-épicatéchine puis la (+)-catéchine, le (+)-gallocatéchine, et (-)-épigallocatéchine ainsi que des proanthocyanidols constitués de 2 ou 3 unités de (+)-catéchol et/ou (-)-épicatéchol, à savoir les procyanidols B1, B2, B3, B4, B5, C1, et D. Les hautes températures de la torréfaction convertissent une partie de la (-)-épicatéchine en son épimère la (-)-catéchine. Les cacaos marchands de la Côte-d'Ivoire contiennent de 2,2 à 4,8 g/kg d'épicatéchine. La fève de cacao contient également des agents tannins.
- les fèves sont riches en catéchines et en agents tannins
- De nombreux fruits sont aussi riches en catéchines et en agents tannins : les mûres en premier, bien sûr le raisin, puis les cerises, abricots, framboises, pommes, prunes, fraises, poires et pêches.

### 3) Fleur de soufre

autre dénomination : CREME DE SOUFRE
autre dénomination : FLEUR DE SOUFRE
bordereau : 2179
sel ou dérivé : SOUFRE COLLOIDAL
sel ou dérivé : SOUFRE PRECIPITE
sel ou dérivé : SOUFRE SUBLIME LAVE
Classes Chimiques Soufre
Constituants : au moins 99.5 pourcents de soufre

### 4) Nigella Sativa (Habbat al Barakah ou Nigelle ou Huile végétale de cumin noir)

### Composition

Acide myristique : 5 %
Acide palmitique : 11.2 - 13.7 %
Acide stéarique : 2.1 - 2.6 %
Acide palmitoléique : 0.1 %
Acide oléique : 20.0 - 23.7 %
Acide linoléique (Omega 6): 57.9 %
Acide alpha-linolénique (Omega 3): 0.2 %
Acide arachidique : 1.3 %

L'huile de Nigelle (ou Nigella ou huile nigelle) est une huile de couleur vert brun d'odeur épicée.

L'huile doit être extraite à froid.

### 5) Menthol

Utilisé sous forme cristalline

### 6) Ethanol

Utilisé pur

### 7) Miel pur

La viscosité du miel doit être importante pour faciliter l'absorption de la substance par l'estomac

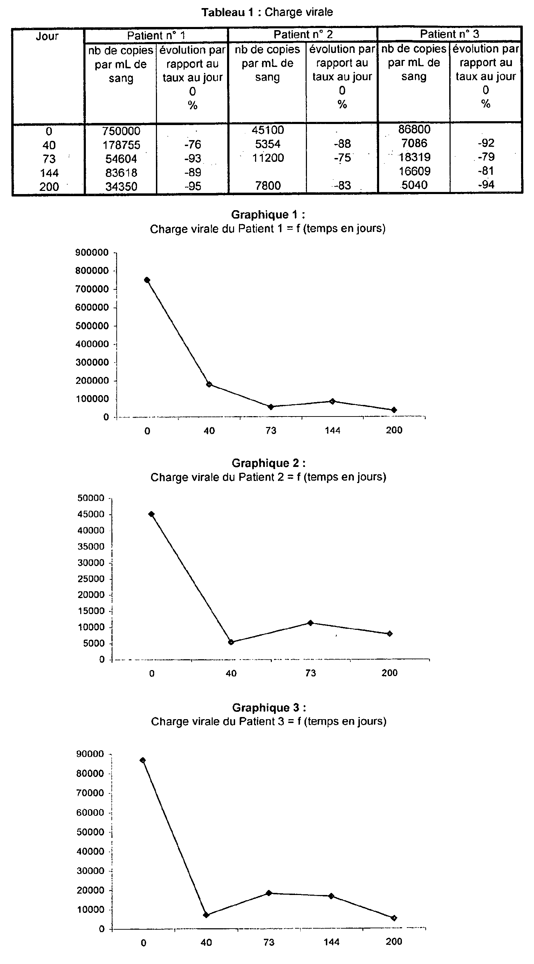

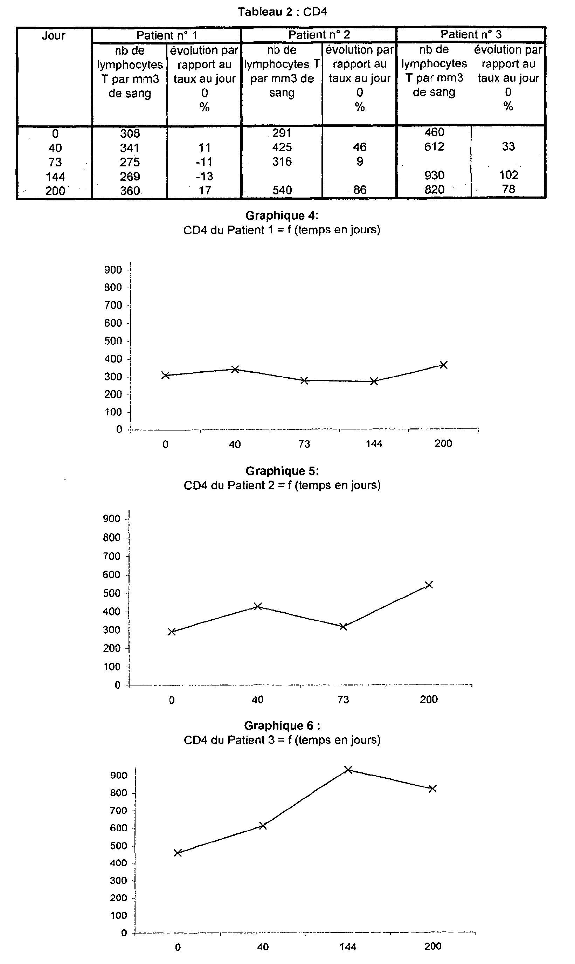

## Revendications

1. Composition à base de plantes, caractérisée en ce qu'elle comprend de la fleur de soufre, au moins une plante contenant des agents tannins et de la catéchine choisie parmi l'Aigremoine Eupatoire (GAFT) ou le gambier (Uncaria gambir), ainsi qu'un support pharmaceutiquement acceptable, pour son utilisation dans le traitement ou la prévention de maladies virales du sang causées par le virus de l'immunodéficience humaine (VIH) ou l'hépatite C.

2. Composition pour son utilisation selon la revendication 1, **caractérisée en ce qu'**elle comprend deux plantes différentes contenant des agents tannins et de la catéchine.

3. Composition pour son utilisation selon la revendication 2, **caractérisée en ce que** les deux plantes différentes contenant des agents tannins et de la catéchine sont l'Aigremoine Eupatoire (GAFT) et du gambier (Uncaria gambir).

4. Composition pour son utilisation selon l'une des revendications précédentes, **caractérisée en ce qu'**elle comprend en outre de la Nigelle.

5. Composition pour son utilisation selon la revendication 4, **caractérisée en ce que** la Nigelle est présente sous la forme d'huile.

6. Composition pour son utilisation selon l'une des revendications précédentes, **caractérisée en ce qu'**elle comprend en outre un agent antiputride.

7. Composition pour son utilisation selon la revendication 6, **caractérisée en ce que** l'agent antiputride est le menthol.

8. Composition pour son utilisation selon l'une des revendications précédentes, **caractérisée en ce qu'**elle comprend un agent d'enrobage.

9. Composition pour son utilisation selon la revendication 8, **caractérisée en ce que** l'agent d'enrobage est le miel.

## Patentansprüche

1. Pflanzliche Zusammensetzung, **dadurch gekennzeichnet, dass** sie Schwefelblüte, mindestens eine Pflanze, die Tanninwirkstoffe und Catechin enthält, ausgewählt aus dem Ackerkraut Agrimonia eupatoria (GAFT) oder dem Gambir (Uncaria gambir), sowie einen pharmazeutisch akzeptablen Trägerstoff umfasst, für ihre Verwendung zur Behandlung oder Prophylaxe von viralen Blutkrankheiten, die von dem humanen Immunschwächevirus (HIV) verursacht werden oder von Hepatitis C.

2. Zusammensetzung für ihre Verwendung nach Anspruch 1, **dadurch gekennzeichnet, dass** sie zwei verschiedene Pflanzen umfasst, die Tanninwirkstoffe und Catechin enthalten.

3. Zusammensetzung für ihre Verwendung nach Anspruch 2, **dadurch gekennzeichnet, dass** die zwei verschiedenen Pflanzen, die Tanninwirkstoffe und Catechin enthalten, das Ackerkraut Agrimonia eupatoria (GAFT) und Gambir (Uncaria gambir) sind.

4. Zusammensetzung für ihre Verwendung nach einem der vorangehenden Ansprüche, **dadurch gekennzeichnet, dass** sie ferner Schwarzkümmel umfasst.

5. Zusammensetzung für ihre Verwendung nach Anspruch 4, **dadurch gekennzeichnet, dass** der Schwarzkümmel in Form von Öl vorhanden ist.

6. Zusammensetzung für ihre Verwendung nach einem der vorangehenden Ansprüche, **dadurch gekennzeichnet, dass** sie ferner ein fäulnishemmendes Mittel umfasst.

7. Zusammensetzung für ihre Verwendung nach Anspruch 6, **dadurch gekennzeichnet, dass** das fäulnishemmende Mittel das Menthol ist.

8. Zusammensetzung für ihre Verwendung nach einem der vorangehenden Ansprüche, **dadurch gekennzeichnet, dass** sie ein Überzugmittel umfasst.

9. Zusammensetzung für ihre Verwendung nach Anspruch 8, **dadurch gekennzeichnet, dass** das Überzugmittel der Honig ist.

## Claims

1. A plant-based composition **characterized in that** it comprises flowers of sulphur, at least one plant containing tannin agents and catechin chosen from among common agrimony (*Agrimonia Eupatoria*, GAFT) or gambier (*Uncaria gambir*), and a pharmaceutically acceptable carrier for use thereof in the treatment or prevention of blood-borne viral diseases caused by the human immunodeficiency virus (HIV) or hepatitis C.

2. The composition for use thereof according to claim 1, **characterized in that** it comprises two different plants containing tannin agents and catechin.

3. The composition for use thereof according to claim 2, **characterized in that** the two different plants containing tannin agents and catechin are common agrimony (*Agrimonia Eupatoria*, GAFT) and gambier (*Uncaria gambir*).

4. The composition for use thereof according to one of the preceding claims, **characterized in that** it further comprises *Nigella.*

5. The composition for use thereof according to claim 4, **characterized in that** the *Nigella* is contained in oil form.

6. The composition for use thereof according to one of the preceding claims, **characterized in that** it further comprises an anti-putrid agent.

7. The composition for use thereof according to claim 6, **characterized in that** the anti-putrid agent is menthol.

8. The composition for use thereof according to one of the preceding claims, characterized that it comprises a coating agent.

9. The composition for use thereof according to claim 8, **characterized in that** the coating agent is honey.
